# EUROPEAN PATENT SPECIFICATION

(11) **EP 3 416 684 B1**
(45) Date of publication and mention of the grant of the patent: **21.06.2023**
(21) Application number: 17706945.7
(22) Date of filing: 13.02.2017
(51) Int. Cl.: A61K 39/395, C07K 16/22, A61P 3/06

(54) **METHODS FOR TREATING OR PREVENTING ATHEROSCLEROSIS BY ADMINISTERING AN INHIBITOR OF ANGPTL3**
VERFAHREN ZUR BEHANDLUNG ODER VORBEUGUNG VON ATHEROSKLEROSE DURCH VERABREICHUNG EINES HEMMERS VON ANGPTL3
PROCÉDÉS DE TRAITEMENT OU DE PRÉVENTION DE L'ATHÉROSCLÉROSE PAR ADMINISTRATION D'UN INHIBITEUR D'ANGPTL3

(30) Priority: 17.02.2016 US 201662296110 P
(43) Date of publication of application: 26.12.2018
(60) Divisional application: 23170382.8
(73) Proprietor: Regeneron Pharmaceuticals, Inc., Tarrytown, NY 10591 (US)
(72) Inventor: GROMADA, Jesper, Tarrytown, New York 10591 (US); GUSAROVA, Viktoria, Tarrytown, New York 10591 (US); MURPHY, Andrew J., Tarrytown, New York 10591 (US)
(74) Representative: J A Kemp LLP
(86) International application number: PCT/US2017/017640
(87) International publication number: WO 2017/142832

(56) References cited:
- WO-A1-2012/174178
- WO-A2-2008/073300
- WO-A2-2011/085271
- US-B2- 8 742 075
- VIKTORIA GUSAROVA ET AL: "ANGPTL3 blockade with a human monoclonal antibody reduces plasma lipids in dyslipidemic mice and monkeys", JOURNAL OF LIPID RESEARCH, vol. 56, no. 7, 29 July 2015 (2015-07-29), pages 1308-1317, XP055363055, US ISSN: 0022-2275, DOI: 10.1194/jlr.M054890
- S. KUHNAST ET AL: "Alirocumab inhibits atherosclerosis, improves the plaque morphology, and enhances the effects of a statin", JOURNAL OF LIPID RESEARCH, vol. 55, no. 10, 1 October 2014 (2014-10-01), pages 2103-2112, XP055363059, US ISSN: 0022-2275, DOI: 10.1194/jlr.M051326
- KASTELEIN JOHN J P ET AL: "Simvastatin with or without ezetimibe in familial hypercholesterolemia.", THE NEW ENGLAND JOURNAL OF MEDICINE 03 APR 2008, vol. 358, no. 14, 3 April 2008 (2008-04-03), pages 1431-1443, ISSN: 1533-4406
- BROWN B GREG ET AL: "Does ENHANCE diminish confidence in lowering LDL or in ezetimibe?", THE NEW ENGLAND JOURNAL OF MEDICINE 03 APR 2008, vol. 358, no. 14, 3 April 2008 (2008-04-03), pages 1504-1507, ISSN: 1533-4406
- ILYAS IQRA ET AL: "Mouse models of atherosclerosis in translational research", TRENDS IN PHARMACOLOGICAL SCIENCES, ELSEVIER, HAYWARTH, GB, vol. 43, no. 11, 25 July 2022 (2022-07-25) , pages 920-939, XP087198057, ISSN: 0165-6147, DOI: 10.1016/J.TIPS.2022.06.009 [retrieved on 2022-07-25]
- REESKAMP LAURENS F ET AL: "Marked plaque regression in homozygous familial hypercholesterolemia", ATHEROSCLEROSIS, ELSEVIER, AMSTERDAM, NL, vol. 327, 3 May 2021 (2021-05-03), pages 13-17, XP086615170, ISSN: 0021-9150, DOI: 10.1016/J.ATHEROSCLEROSIS.2021.04.014 [retrieved on 2021-05-03]

## Description

### FIELD OF THE INVENTION

The present invention relates to the use of ANGPTL3 inhibitors to reduce the progression of atherosclerosis.

### BACKGROUND

Atherosclerosis is a syndrome affecting arterial blood vessels. Atherosclerosis results from a chronic inflammatory response in the arterial walls and is a major cause of death and cardiovascular morbidity. Atherosclerosis involves the thickening of artery walls and is characterized by the development of arterial plaque, and the eventual restriction or blockage of blood flow. Current atherosclerosis treatments include diet and lifestyle modifications (*e.g*. smoking cessation), pharmaceutical intervention (*e.g*., statins, anti-coagulants, etc.), and surgery (*e.g*., angioplasty, stents, bypass surgery, etc.). Direct evaluation of the efficacy of pharmaceutical interventions on atherosclerosis development in human subjects is problematic due to the difficulty in accurately detecting and measuring plaque and arterial lesions in live subjects. Animal models of atherosclerosis are therefore very useful for the development and testing of novel therapeutic treatments for atherosclerosis.

New methods for the treatment, prevention or amelioration of atherosclerosis are needed in the art.

WO 2012/174178 discloses a fully human antibody of antigen-binding fragment of a human antibody that specifically binds and inhibits or interferes with at least one activity of human angiopoietin-like protein 3 (hANGPTL3).. WO 2011/085271 discloses methods compounds and compositions for reducing expression of an ANGPTL3 mRNA and protein in an animal.

### BRIEF SUMMARY OF THE INVENTION

Any references in the description to methods of treatment refer to the compounds, pharmaceutical compositions and medicaments of the present invention for use in a method for treatment of the human (or animal) body by therapy. The present invention provides an angiopoietin-like protein 3 (ANGPTL3) inhibitor for use in a method of reducing the progression of atherosclerosis in a subject, the method comprising selecting a subject who has atherosclerosis and administering one or more doses of the ANGPTL3 inhibitor to the subject, wherein the ANGPTL3 inhibitor is an antibody or antigen-binding fragment thereof that specifically binds ANGPTL3 which comprises heavy and light chain CDR amino acid sequences having SEQ ID NOs:4, 5, 6, 7, 8, and 9. The therapeutic methods may result in a reduction in atherosclerotic plaque and atherosclerotic lesion formation in subjects in need thereof. The methods are also useful for reducing serum total cholesterol, LDL cholesterol, and triglycerides in a subject, and for treating diseases and disorders that are treatable by a reduction in one or more of total cholesterol, LDL cholesterol and/or triglycerides.

According to certain embodiments of the present invention, the subject, prior to or at the time of initiation of treatment with the one or more doses of the ANGPTL3 inhibitor, is diagnosed with heterozygous familial hypercholesterolemia (HeFH) or homozygous familial hypercholesterolemia (HoFH), and/or elevated lipoprotein(a) (Lp[a]). The present invention also includes an ANGPTL3 inhibitor as defined in the claims for use in methods of reducing the progression of atherosclerosis in a subject who has hypercholesterolemia that is not familial in nature (*i.e.*, non-familial hypercholesterolemia). Subjects who are treatable by the methods, in certain embodiments, include those subjects who have or are diagnosed with cardiovascular disease (e.g., atherosclerotic cardiovascular disease). In certain embodiments, the subject who is treatable is a subject who has suffered a stroke or myocardial infarction.

According to certain embodiments of the present invention, the ANGPTL3 inhibitor is administered to the patient as an add-on therapy to the patient's existing lipid-modifying therapy (*e.g.,* on top of the patient's background statin therapy).

Other embodiments of the present invention will become apparent from a review of the ensuing detailed description.

### BRIEF DESCRIPTION OF THE FIGURES

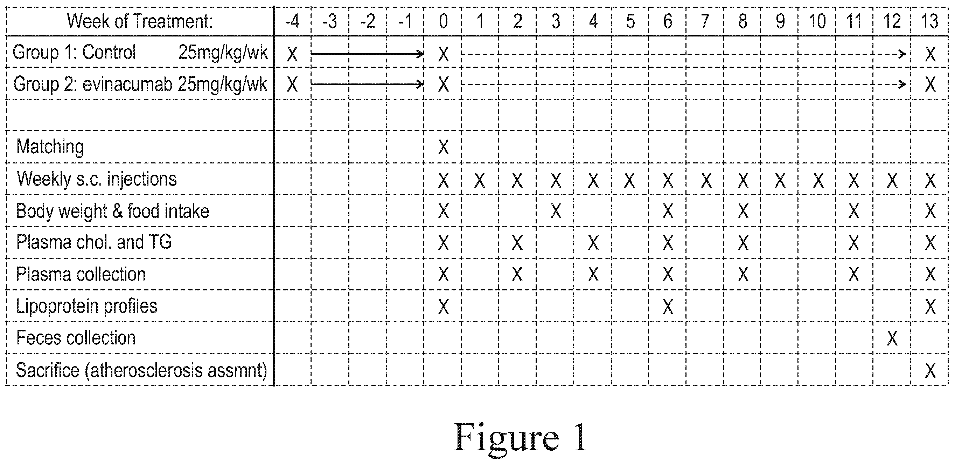

**Figure 1** is a study flow diagram illustrating the procedures, administrations and measurements performed on the animals throughout the course of the study described in Example 2 herein.

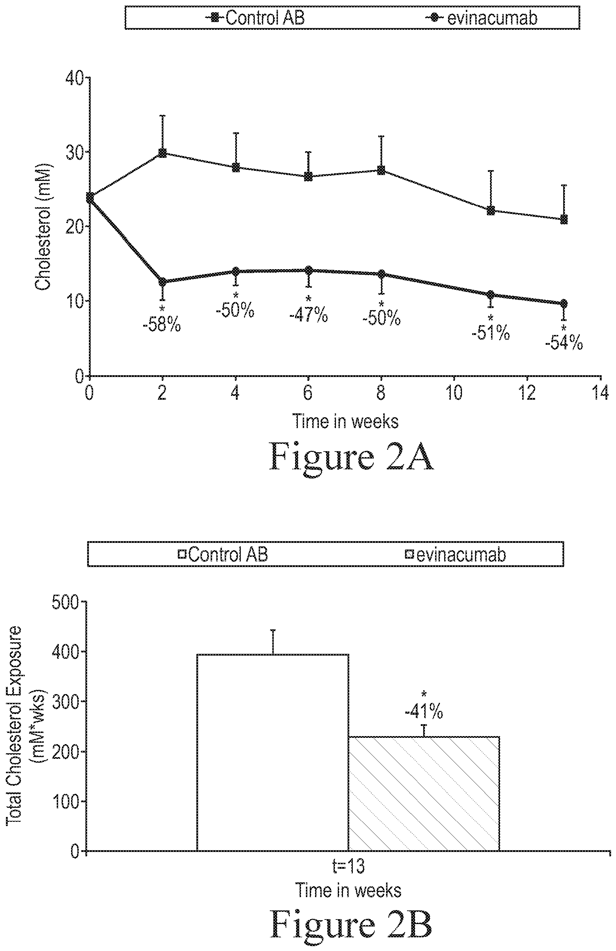

**Figures 2A** and **2B** show the cholesterol levels of animals from the different treatment groups (control treated, closed squares [■]; or evinacumab-treated, closed circles [●]) at different time points throughout the course of the study described in Example 2 herein. **Figure 2A** shows mean cholesterol levels (mM) over time (in weeks). **Figure 2B** shows total cholesterol exposure (mM x wks) at week 13.

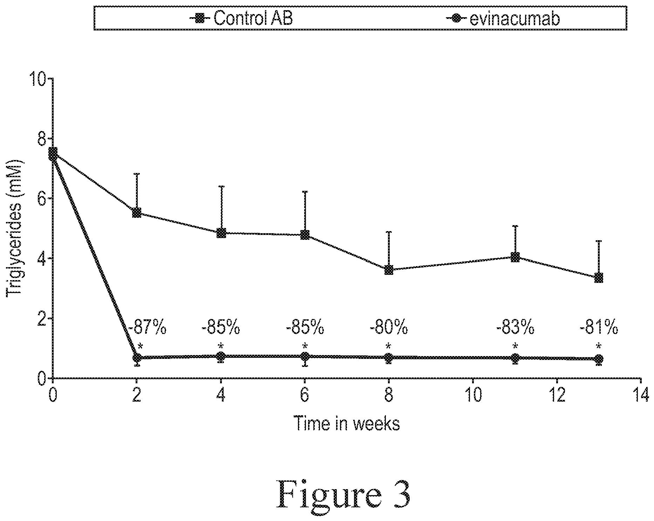

**Figure 3** shows the triglyceride levels (mM) of animals from the different treatment groups (control treated, closed squares [■]; or evinacumab-treated, closed circles [●]) at different time points throughout the course of the study described in Example 2 herein.

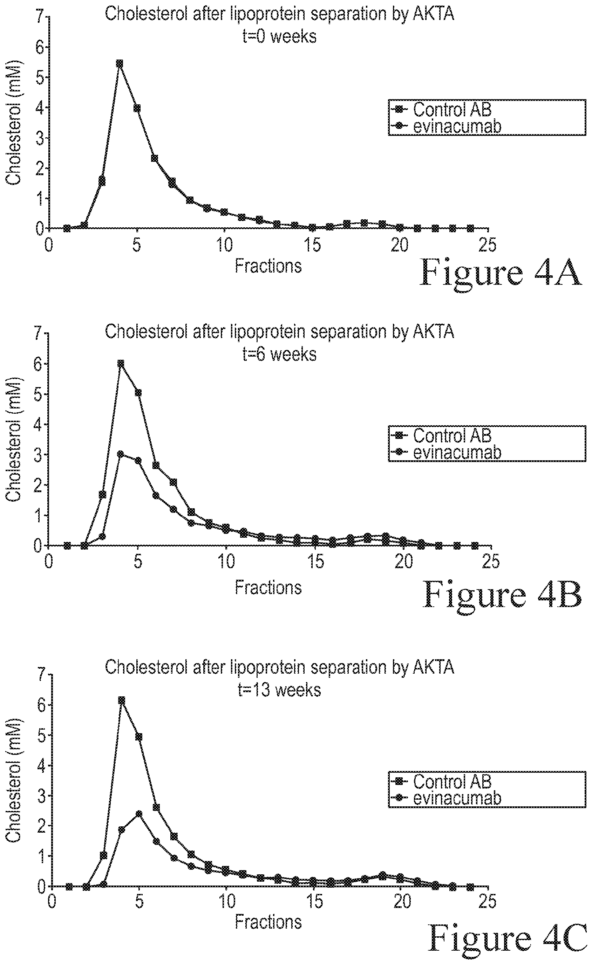

**Figures 4A, 4B,** and **4C** show the cholesterol lipoprotein profiles (mM, after lipoprotein separation) of animals from the different treatment groups (control treated, closed squares [■]; or evinacumab-treated, closed circles [●]) at different time points throughout the course of the study described in Example 2 herein. Lipoproteins were separated on a Superose column, and the values shown are absolute values from cholesterol measurements in pooled plasma per group at the various time points indicated. **Figure 4A** shows the cholesterol levels from different fractions of plasma drawn at time=0 of the study. **Figure 4B** shows the cholesterol levels from different fractions of plasma drawn at time=6 weeks of the study. **Figure 4C** shows the cholesterol levels from different fractions of plasma drawn at time=13 weeks of the study.

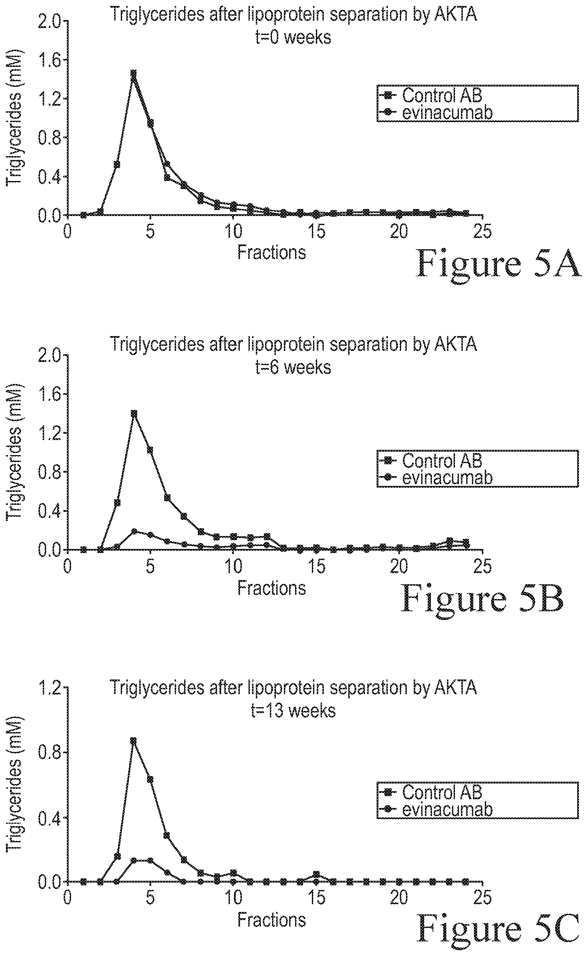

**Figures 5A, 5B,** and **5C** show the triglyceride lipoprotein profiles (mM, after lipoprotein separation) of animals from the different treatment groups (control treated, closed squares [■]; or evinacumab-treated, closed circles [●]) at different time points throughout the course of the study described in Example 2 herein. Lipoproteins were separated on a Superose column, and the values shown are absolute values from triglyceride measurements in pooled plasma per group at the various time points indicated. **Figure 5A** shows the triglyceride levels from different fractions of plasma drawn at time=0 of the study. **Figure 5B** shows the triglyceride levels from different fractions of plasma drawn at time=6 weeks of the study. **Figure 5C** shows the triglyceride levels from different fractions of plasma drawn at time=13 weeks of the study.

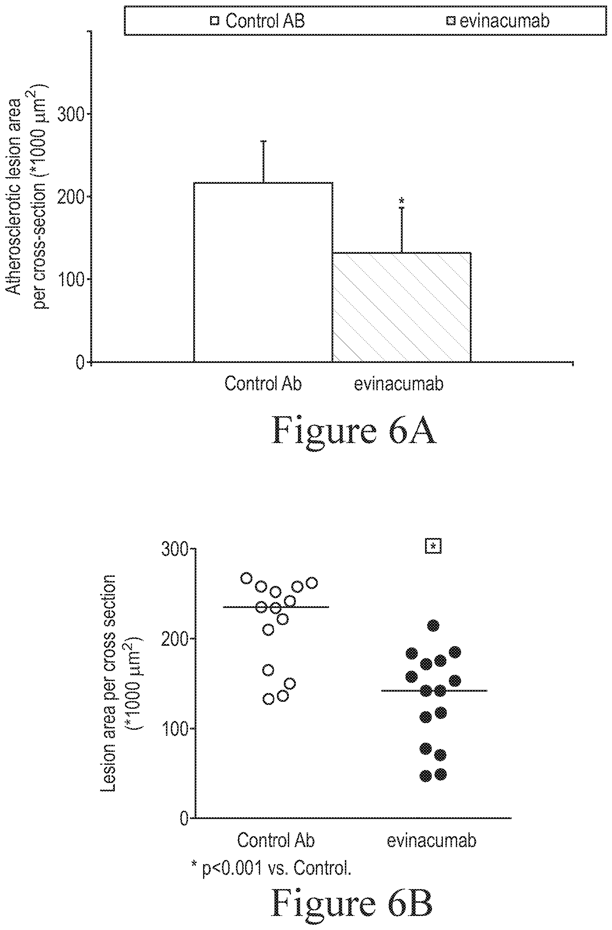

**Figures 6A** and **6B** show the atherosclerotic lesion area of animals from the different treatment groups (control treated, open bar or circles; or evinacumab-treated, closed bar or circles) at week 13 of the study described in Example 2 herein. **Figure 6A** shows the mean atherosclerotic lesion area per cross-section (x1000 µm²) for the different treatment groups. **Figure 6B** is a scatter plot of total lesion area, showing the atherosclerotic lesion area per cross-section (x1000 µm²) for individual animals in the different treatment groups.

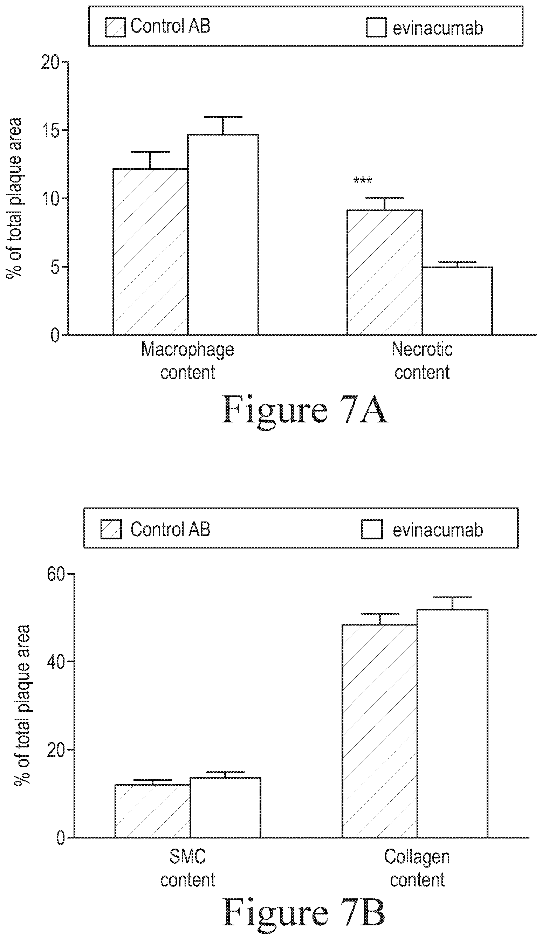

**Figures 7A** and **7B** show the mean necrotic content of cross-sections from atherosclerotic lesions, categorized as type IV or type V, observed in animals from the different treatment groups (control treated, open bar; or evinacumab-treated, closed bar) at week 13 of the study described in Example 2 herein. **Figure 7A** shows the absolute value (µm²×1000) of necrotic area per cross-section. **Figure 7B** shows relative values (percent of lesion area) of necrotic content of the lesions.

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 1%. For example, as used herein, the expression "about 100" includes 99 and 101 and all values in between (*e.g.*, 99.1, 99.2, 99.3, 99.4, etc.).

Although any methods and materials similar or equivalent to those described herein can be used in the practice of the present invention, the preferred methods and materials are now described..

### Methods for Reducing Progression of Atherosclerosis

The present invention relates generally to the use of an angiopoietin-like protein-3 (ANGPTL3) inhibitor as defined in the claims for reducing progression of atherosclerosis in a subject. An angiopoietin-like protein-3 (ANGPTL3) inhibitor as defined in the claims is used in one or more doses. According to certain embodiments, the use an angiopoietin-like protein-3 (ANGPTL3) inhibitor as defined in the claims results in a reduction in atherosclerotic plaque formation in a subject. For example, the use of an angiopoietin-like protein-3 (ANGPTL3) inhibitor as defined in the claims may result in one or more of a reduction in atherosclerotic lesion area and/or a reduction in necrotic area of atherosclerotic lesions in a subject.

### Patient Selection

According to the present invention, subjects who are treatable by the methods are subjects who have or are diagnosed with atherosclerosis. Accordingly, the methods include selecting a subject having atherosclerosis, and administering one or more doses of the angiopoietin-like protein-3 (ANGPTL3) inhibitor to the subject.

According to certain embodiments of the present invention, the subject who is treatable by the methods is a subject who, prior to or at the time of initiation of treatment with the one or more doses of the ANGPTL3 inhibitor, is diagnosed with heterozygous familial hypercholesterolemia (HeFH), homozygous familial hypercholesterolemia (HoFH), elevated lipoprotein(a) (Lp[a]), Autosomal Dominant Hypercholesterolemia (ADH, *e.g.*, ADH associated with one or more gain-of-function mutations in the LDLR gene, the ApoB gene, and/or the PCSK9 gene), autosomal recessive hypercholesterolemia (ARH, *e.g*., ARH associated with mutations in LDLRAP1), as well as incidences of hypercholesterolemia that are distinct from Familial Hypercholesterolemia (nonFH). Diagnosis of familial hypercholesterolemia (*e.g*., heFH or hoFH) can be made by genotyping and/or clinical criteria. For patients who are not genotyped, clinical diagnosis may be based on either the Simon Broome criteria with a criteria for definite FH, or the WHO/Dutch Lipid Network criteria with a score > 8 points.

According to certain embodiments of the present invention, the subject who is treatable by the methods is a subject who is diagnosed with cardiovascular disease (CVD) prior to or at the time of initiation of treatment with the one or more doses of the ANGPTL3 inhibitor. The subject may also be selected on the basis of having a history of coronary heart disease (CHD). As used herein a "history of CHD" (or "documented history of CHD") includes one or more of: (i) acute myocardial infarction (MI); (ii) silent MI; (iii) unstable angina; (iv) coronary revascularization procedure (e.g., percutaneous coronary intervention [PCI] or coronary artery bypass graft surgery [CABG]); and/or (v) clinically significant CHD diagnosed by invasive or non-invasive testing (such as coronary angiography, stress test using treadmill, stress echocardiography or nuclear imaging).

According to certain embodiments of the present invention, the subject who is treatable by the methods may be selected on the basis of having non-coronary heart disease cardiovascular disease ("non-CHD CVD"). As used herein, "non-CHD CVD" includes one or more of: (i) documented previous ischemic stroke with a focal ischemic neurological deficit that persisted more than 24 hours, considered as being of atherothrombotic origin; (ii) peripheral arterial disease; (iii) abdominal aortic aneurysm; (iv) atherosclerotic renal artery stenosis; and/or (v) carotid artery disease (transient ischemic attacks or >50% obstruction of a carotid artery).

According to certain embodiments of the present invention, the subject who is treatable by the methods may be selected on the basis of having one or more additional risk factors such as, *e.g.,* (i) documented moderate chronic kidney disease (CKD) as defined by 30 ≤eGFR <60 mUmin/1.73 m2 for 3 months or more; (ii) type 1 or type 2 diabetes mellitus with or without target organ damage (*e.g*., retinopathy, nephropathy, microalbuminuria); and/or (iii) a calculated 10-year fatal CVD risk SCORE ≥5% (ESC/EAS Guidelines for the management of dyslipidemias, Conroy et al., 2003, Eur. Heart J. 24:987-1003).

According to certain embodiments of the present invention, the subject who is treatable by the methods may be selected on the basis of having one or more additional risk factors selected from the group consisting of age (*e.g*., older than 40, 45, 50, 55, 60, 65, 70, 75, or 80 years), race, national origin, gender (male or female), exercise habits (*e.g*., regular exerciser, non-exerciser), other preexisting medical conditions (*e.g.*, type-II diabetes, high blood pressure, etc.), and current medication status (*e.g*., currently taking beta blockers, niacin, ezetimibe, fibrates, omega-3 fatty acids, bile acid resins, etc.).

According to certain embodiments of the present invention, the subject who is treatable by the methods exhibits an elevated level of one or more inflammatory marker. Any marker of systemic inflammation can be utilized for the purposes of the present invention. Suitable inflammatory markers include, without limitation, C-reactive protein, cytokines (*e.g*., II-6, IL-8, and/or IL-17), and cellular adhesion molecules (*e.g*., ICAM-1, ICAM-3, BL-CAM, LFA-2, VCAM-1, NCAM, and PECAM).

According to certain embodiments of the present invention, the subject who is treatable by the methods may be selected on the basis of a combination of one or more of the foregoing selection criteria or therapeutic characteristics. For example, according to certain embodiments, a subject suitable for treatment with the methods may further be selected on the basis of having heFH or non-FH in combination with: (i) a history of documented CHD, (ii) non-CHD CVD, and/or (iii) diabetes mellitus with target organ damage; such patients may also be selected on the basis of having a serum LDL-C concentration of greater than or equal to 70 mg/dL.

According to certain embodiments of the present invention, the subject who is treatable by the methods has hypercholesterolemia that is not adequately controlled by a daily moderate-dose therapeutic statin regimen, and may further be selected on the basis of having heFH or non-FH without CHD, or non-CHD CVD, but having either (i) a calculated 10-year fatal CVD risk SCORE ≥5%; or (ii) diabetes mellitus without target organ damage; such subjects may also be selected on the basis of having a serum LDL-C concentration of greater than or equal to 100 mg/dL.

According to certain embodiments of the present invention, the subject who is treatable by the methods is a subject who has familial chylomicronemia syndrome (FCS; also known as lipoprotein lipase deficiency).

According to certain embodiments of the present invention, the subject who is treatable by the methods is a subject who is undergoing, or has recently undergone, lipoprotein apheresis (e.g., within the last six months, within the last 12 weeks, within the last 8 weeks, within the last 6 weeks, within the last 4 weeks, within the last 2 weeks, etc.).

### Administration of an ANGPTL3 Inhibitor as Add-On Therapy

The present invention includes an ANGPTL3 inhibitor as defined in the claims for use in methods of reducing the progression of atherosclerosis in a subject, wherein the subject is administered the ANGPTL3 inhibitor according to a particular dosing amount and frequency, and wherein the ANGPTL3 inhibitor is administered as an add-on to the patient's pre-existing lipid modifying therapy (LMT), such as an add-on to the patient's pre-existing daily therapeutic statin regimen. (Specific, exemplary daily therapeutic statin regimens to which an ANGPTL3 inhibitor may be added are described elsewhere herein).

For example, the methods include add-on therapeutic regimens wherein the ANGPTL3 inhibitor is administered as add-on therapy to the same stable daily therapeutic statin regimen (i.e., same dosing amount of statin) that the patient was on prior to receiving the ANGPTL3 inhibitor. In other embodiments, the ANGPTL3 inhibitor is administered as add-on therapy to a therapeutic statin regimen comprising a statin in an amount that is more than or less than the dose of stain the patient was on prior to receiving the ANGPTL3 inhibitor. For example, after starting a therapeutic regimen comprising an ANGPTL3 inhibitor administered at a particular dosing frequency and amount, the daily dose of statin administered or prescribed to the patient may (a) stay the same, (b) increase, or (c) decrease (e.g., up-titrate or down-titrate) in comparison to the daily statin dose the patient was taking before starting the ANGPTL3 inhibitor therapeutic regimen, depending on the therapeutic needs of the patient.

### Therapeutic Efficacy

The methods result in a reduction in atherosclerotic plaque formation in the arteries of a subject, and/or the prevention atherosclerosis progression in a subject. For example, the methods are useful for limiting or reducing atherosclerotic lesion area and/or necrotic core area in atherosclerotic lesions in a subject.

The methods may additionally result in the reduction in serum levels of one or more lipid component selected from the group consisting of LDL-C, ApoB100, non-HDL-C, total cholesterol, VLDL-C, triglycerides, Lp(a) and remnant cholesterol. For example, according to certain embodiments of the present invention, administration of a pharmaceutical composition comprising an ANGPTL3 inhibitor to a suitable subject will result in a mean percent reduction from baseline in serum low density lipoprotein cholesterol (LDL-C) of at least about 25%, 30%, 40%, 50%, 60%, or greater; a mean percent reduction from baseline in ApoB100 of at least about 25%, 30%, 40%, 50%, 60%, or greater; a mean percent reduction from baseline in non-HDL-C of at least about 25%, 30%, 40%, 50%, 60%, or greater; a mean percent reduction from baseline in total cholesterol of at least about 10%, 15%, 20%, 25%, 30%, 35%, or greater; a mean percent reduction from baseline in VLDL-C of at least about 5%, 10%, 15%, 20%, 25%, 30%, or greater; a mean percent reduction from baseline in triglycerides of at least about 5%, 10%, 15%, 20%, 25%, 30%, 35% or greater; and/or a mean percent reduction from baseline in Lp(a) of at least about 5%, 10%, 15%, 20%, 25%, or greater.

### ANGPTL3 Inhibitors

The methods of the present invention comprise administering to a patient a therapeutic composition comprising an ANGPTL3 inhibitor. In the context of the present invention, ANPTL3 inhibitors are anti-ANGPTL3 antibodies or antigen-binding fragments of antibodies that specifically bind human ANGPTL3 which comprise heavy and light chain CDR amino acid sequences having SEQ ID NOs:4, 5, 6, 7, 8, and 9.

The term "human angiopoietin-like protein-3" or "human ANGPTL3" or "hANGPTL3", as used herein, refers to ANGPTL3 having the amino acid sequence of SEQ ID NO:1 (see also NCBI Accession NP_055310), or a biologically active fragment thereof.

The term "antibody", as used herein, is intended to refer to immunoglobulin molecules comprising four polypeptide chains, two heavy (H) chains and two light (L) chains interconnected by disulfide bonds, as well as multimers thereof (*e.g.,* IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (C_{L}1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different embodiments of the invention, the FRs of the anti-ANGPTL3 antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g., commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (*e.g*., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (*e.g*. monovalent nanobodies, bivalent nanobodies, etc.), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

In certain aspects of the disclosure, an antigen-binding fragment of an antibody may contain at least one variable domain covalently linked to at least one constant domain. Non-limiting, exemplary configurations of variable and constant domains that may be found within an antigen-binding fragment of an antibody include: (i) V_{H}-C_{H}1 ; (ii) V_{H}-C_{H}2; (iii) V_{H}-C_{H}3; (iv) V_{H}-C_{H}1-C_{H}2; (v) V_{H}-C_{H}1-C_{H}2-C_{H}3; (vi) V_{H}-C_{H}2-C_{H}3; (vii) V_{H}-C_{L}; (viii) V_{L}-C_{H}1; (ix) V_{L}-C_{H}2; (x) V_{L}-C_{H}3; (xi) V_{L}-C_{H}1-C_{H}2; (xii) V_{L}-C_{H}1-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (*e.g.,* 5, 10, 15, 20, 40, 60 or more) amino acids which result in a flexible or semi-flexible linkage between adjacent variable and/or constant domains in a single polypeptide molecule. Moreover, an antigen-binding fragment of an antibody may comprise a homo-dimer or hetero-dimer (or other multimer) of any of the variable and constant domain configurations listed above in noncovalent association with one another and/or with one or more monomeric V_{H} or V_{L} domain (*e.g.,* by disulfide bond(s)).

As with full antibody molecules, antigen-binding fragments may be monospecific or multispecific (*e.g*., bispecific). A multispecific antigen-binding fragment of an antibody will typically comprise at least two different variable domains, wherein each variable domain is capable of specifically binding to a separate antigen or to a different epitope on the same antigen. Any multispecific antibody format, including the exemplary bispecific antibody formats disclosed herein, may be adapted for use in the context of an antigen-binding fragment of an antibody using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody", as used herein, is intended to include antibodies having variable and constant regions derived from human germline immunoglobulin sequences. The human antibodies may nonetheless include amino acid residues not encoded by human germline immunoglobulin sequences (e.g., mutations introduced by random or site-specific mutagenesis *in vitro* or by somatic mutation *in vivo*)*,* for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

Human antibodies can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via inter-chain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. The instant invention encompasses antibodies having one or more mutations in the hinge, C_{H}2 or C_{H}3 region which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present invention. An isolated antibody also includes an antibody *in situ* within a recombinant cell. Isolated antibodies are antibodies that have been subjected to at least one purification or isolation step. According to certain embodiments, an isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" ANGPTL3, as used in the context of the present disclosure, includes antibodies that bind ANGPTL3 or portion thereof with a K_{D} of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or less than about 0.5 nM, as measured in a surface plasmon resonance assay. An isolated antibody that specifically binds human ANGPTL3, however, has cross-reactivity to other antigens, such as ANGPTL3 molecules from other (non-human) species.

The anti-ANGPTL3 antibodies of the present disclosure may comprise one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases. The present disclosure includes the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). A person of ordinary skill in the art, starting with the heavy and light chain variable region sequences disclosed herein, can easily produce numerous antibodies and antigen-binding fragments which comprise one or more individual germline mutations or combinations thereof. In certain aspects of the disclosure, all of the framework and/or CDR residues within the V_{H} and/or V_{L} domains are mutated back to the residues found in the original germline sequence from which the antibody was derived. In other aspects of the disclosure, only certain residues are mutated back to the original germline sequence, e.g., only the mutated residues found within the first 8 amino acids of FR1 or within the last 8 amino acids of FR4, or only the mutated residues found within CDR1, CDR2 or CDR3. In other aspects, one or more of the framework and/or CDR residue(s) are mutated to the corresponding residue(s) of a different germline sequence (i.e., a germline sequence that is different from the germline sequence from which the antibody was originally derived). Furthermore, the antibodies may contain any combination of two or more germline mutations within the framework and/or CDR regions, e.g., wherein certain individual residues are mutated to the corresponding residue of a particular germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of antibodies and antigen-binding fragments obtained in this general manner are encompassed within the present disclosure.

The present disclosure also includes use of anti-ANGPTL3 antibodies comprising variants of any of the HCVR, LCVR, and/or CDR amino acid sequences disclosed herein having one or more conservative substitutions. For example, the present disclosure includes the use of anti-ANGPTL3 antibodies having HCVR, LCVR, and/or CDR amino acid sequences with, *e.g*., 10 or fewer, 8 or fewer, 6 or fewer, 4 or fewer, etc. conservative amino acid substitutions relative to any of the HCVR, LCVR, and/or CDR amino acid sequences disclosed herein.

The term "surface plasmon resonance", as used herein, refers to an optical phenomenon that allows for the analysis of real-time interactions by detection of alterations in protein concentrations within a biosensor matrix, for example using the BIAcore^{™} system (Biacore Life Sciences division of GE Healthcare, Piscataway, NJ).

The term "K_{D} ", as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

According to certain embodiments, the anti-ANGPTL3 antibody used in the present invention is an antibody with pH-dependent binding characteristics. As used herein, the expression "pH-dependent binding" means that the antibody or antigen-binding fragment thereof exhibits "reduced binding to ANGPTL3 at acidic pH as compared to neutral pH" (for purposes of the present disclosure, both expressions may be used interchangeably). For the example, antibodies "with pH-dependent binding characteristics" include antibodies and antigen-binding fragments thereof that bind ANGPTL3 with higher affinity at neutral pH than at acidic pH. In certain embodiments, the antibodies and antigen-binding fragments bind ANGPTL3 with at least 3, 5, 10, 15, 20, 25, 30, 35, 40, 45, 50, 55, 60, 65, 70, 75, 80, 85, 90, 95, 100, or more times higher affinity at neutral pH than at acidic pH.

According to this aspect of the invention, the anti-ANGPTL3 antibodies with pH-dependent binding characteristics may possess one or more amino acid variations relative to the parental anti-ANGPTL3 antibody. For example, an anti-ANGPTL3 antibody with pH-dependent binding characteristics may contain one or more histidine substitutions or insertions, *e.g.,* in one or more CDRs of a parental anti-ANGPTL3 antibody. Thus, according to certain aspects of the disclosure, methods are provided comprising administering an anti-ANGPTL3 antibody which comprises CDR amino acid sequences (*e.g*., heavy and light chain CDRs) which are identical to the CDR amino acid sequences of a parental anti-ANGPTL3 antibody, except for the substitution of one or more amino acids of one or more CDRs of the parental antibody with a histidine residue. The anti-ANGPTL3 antibodies with pH-dependent binding may possess, *e.g*., 1, 2, 3, 4, 5, 6, 7, 8, 9, or more histidine substitutions, either within a single CDR of a parental antibody or distributed throughout multiple (*e.g*., 2, 3, 4, 5, or 6) CDRs of a parental anti-ANGPTL3 antibody. For example, the present disclosure includes the use of anti-ANGPTL3 antibodies with pH-dependent binding comprising one or more histidine substitutions in HCDR1, one or more histidine substitutions in HCDR2, one or more histidine substitutions in HCDR3, one or more histidine substitutions in LCDR1, one or more histidine substitutions in LCDR2, and/or one or more histidine substitutions in LCDR3, of a parental anti-ANGPTL3 antibody.

As used herein, the expression "acidic pH" means a pH of 6.0 or less (*e.g*., less than about 6.0, less than about 5.5, less than about 5.0, etc.). The expression "acidic pH" includes pH values of about 6.0, 5.95, 5.90, 5.85, 5.8, 5.75, 5.7, 5.65, 5.6, 5.55, 5.5, 5.45, 5.4, 5.35, 5.3, 5.25, 5.2, 5.15, 5.1, 5.05, 5.0, or less. As used herein, the expression "neutral pH" means a pH of about 7.0 to about 7.4. The expression "neutral pH" includes pH values of about 7.0, 7.05, 7.1, 7.15, 7.2, 7.25, 7.3, 7.35, and 7.4.

Non-limiting examples of anti-ANGPTL3 antibodies that can be used in the context of the present disclosure include, *e.g*., evinacumab, as well as any of the exemplary anti-ANGPTL3 antibodies as set forth in US Patent No. 9,018,356 (Regeneron Pharmaceuticals, Inc.), or any of the exemplary anti-ANGPTL3 antibodies as set forth in US Patent No. 8,742,075 (Lexicon Pharmaceuticals, Inc.). In the invention, the antibody may be evinacumab.

### Preparation of Human Antibodies

Anti-ANGPTL3 antibodies can be made according to any method of antibody production/isolation known in the art. For example, antibodies may be made by hybridoma technologies, by phage display, by yeast display, etc. Antibodies for use in the present invention may be, *e.g*., chimeric antibodies, humanized antibodies, or fully human antibodies.

Methods for generating human antibodies in transgenic mice are known in the art. Any such known methods can be used to make human antibodies that specifically bind ANGPTL3.

For example, using VELOCIMMUNE^{™} technology (see, for example, US 6,596,541, Regeneron Pharmaceuticals) or any other known method for generating monoclonal antibodies, high affinity chimeric antibodies to ANGPTL3 are initially isolated having a human variable region and a mouse constant region. The VELOCIMMUNE^{®} technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE^{®} mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc., using standard procedures known to those skilled in the art. The mouse constant regions are replaced with a desired human constant region to generate the fully human antibody of the invention, for example wild-type or modified IgG1 or IgG4. While the constant region selected may vary according to specific use, high affinity antigen-binding and target specificity characteristics reside in the variable region.

In general, the antibodies possess high affinities, as described above, when measured by binding to antigen either immobilized on solid phase or in solution phase. The mouse constant regions are replaced with desired human constant regions to generate the fully human antibodies. While the constant region selected may vary according to specific use, high affinity antigen-binding and target specificity characteristics reside in the variable region.

In the present invention, the anti-ANGPTL3 antibody, or antigen-binding fragment thereof comprises heavy and light chain complementarity determining regions (HCDR1-HCDR2-HCDR3/LCDR1-LCDR2-LCDR3) comprising the amino acid sequences of SEQ ID NOs:4, 5, 6, 7, 8 and 9.

In certain embodiments of the present invention, the anti-ANGPTL3 antibody, or antigen-binding fragment thereof, that can be used in the methods comprises an HCVR having the amino acid sequence of SEQ ID NO:2 and an LCVR having the amino acid sequence of SEQ ID NO:3.

### Pharmaceutical Compositions and Methods of Administration

The present invention includes administering an ANGPTL3 inhibitor as defined in the claims to a patient, wherein the ANGPTL3 inhibitor is contained within a pharmaceutical composition. The pharmaceutical compositions are formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN^{™}), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

Various delivery systems are known and can be used to administer the pharmaceutical composition, *e.g*., encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition can be delivered subcutaneously or intravenously with a standard needle and syringe. In addition, with respect to subcutaneous delivery, a pen delivery device readily has applications in delivering a pharmaceutical composition. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition. Examples include, but are not limited to AUTOPEN^{™} (Owen Mumford, Inc., Woodstock, UK), DISETRONIC^{™} pen (Disetronic Medical Systems, Bergdorf, Switzerland), HUMALOG MIX 75/25^{™} pen, HUMALOG^{™} pen, HUMALIN 70/30^{™} pen (Eli Lilly and Co., Indianapolis, IN), NOVOPEN^{™} I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, NJ), OPTIPEN^{™}, OPTIPEN PRO^{™}, OPTIPEN STARLET^{™}, and OPTICLIK^{™} (Sanofi-Aventis, Frankfurt, Germany), to name only a few. Examples of disposable pen delivery devices having applications in subcutaneous delivery of a pharmaceutical composition of the present invention include, but are not limited to the SOLOSTAR^{™} pen (Sanofi-Aventis), the FLEXPEN^{™} (Novo Nordisk), and the KWIKPEN^{™} (Eli Lilly), the SURECLICK^{™} Autoinjector (Amgen, Thousand Oaks, CA), the PENLET^{™} (Haselmeier, Stuttgart, Germany), the EPIPEN (Dey, L.P.), and the HUMIRA^{™} Pen (Abbott Labs, Abbott Park IL), to name only a few.

In certain situations, the pharmaceutical composition can be delivered in a controlled release system. In one embodiment, a pump may be used (see Langer, supra; Sefton, 1987, CRC Crit. Ref. Biomed. Eng. 14:201). In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the composition's target, thus requiring only a fraction of the systemic dose (see, *e.g*., Goodson, 1984, in Medical Applications of Controlled Release, supra, vol. 2, pp. 115-138). Other controlled release systems are discussed in the review by Langer, 1990, Science 249:1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, *e.g*., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc.

### Dosage

The amount of anti-ANGPTL3 antibody administered to a subject according to the present invention is, generally, a therapeutically effective amount. As used herein, the phrase "therapeutically effective amount" means a dose of ANGPTL3 inhibitor that results in a detectable reduction (at least about 5%, 10%, 15%, 20%, 25%, 30%, 35%, 40%, 45%, 50%, 55%, 60%, 65%, 70%, 75%, or more from baseline) in one or more parameters selected from the group consisting of LDL-C, ApoB100, non-HDL-C, total cholesterol, VLDL-C, triglycerides, Lp(a) and remnant cholesterol, or an amount that prevents or attenuates atherosclerosis in a subject (as described elsewhere herein).

In the case of an anti-ANGPTL3 antibody, a therapeutically effective amount can be from about 0.05 mg to about 600 mg, *e.g*., about 0.05 mg, about 0.1 mg, about 1.0 mg, about 1.5 mg, about 2.0 mg, about 10 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-ANGPTL3 antibody. Other dosing amounts of ANGPTL3 inhibitors will be apparent to persons of ordinary skill in the art.

The amount of anti-ANGPTL3 antibody contained within the individual doses may be expressed in terms of milligrams of antibody per kilogram of patient body weight (*i.e*., mg/kg). For example, the anti-ANGPTL3 antibody may be administered to a patient at a dose of about 0.0001 to about 10 mg/kg of patient body weight.

### Combination Therapies

As described elsewhere herein, the present invention may comprise administering an ANGPTL3 inhibitor to a patient in combination with ("on top of") the patient's previously prescribed lipid lowering therapy. For example, in the context of preventing or attenuating atherosclerosis, an ANGPTL3 inhibitor may be administered to a patient in combination with a stable daily therapeutic statin regimen. Exemplary daily therapeutic statin regimens that an ANGPTL3 inhibitor may be administered in combination with in the context of the present invention include, *e.g*., atorvastatin (10, 20, 40 or 80 mg daily), (atorvastatin/ezetimibe 10/10 or 40/10 mg daily), rosuvastatin (5, 10 or 20 mg daily), cerivastatin (0.4 or 0.8 mg daily), pitavastatin (1, 2 or 4 mg daily), fluvastatin (20, 40 or 80 mg daily), simvastatin (5, 10, 20, 40 or 80 mg daily), simvastatin/ezetimibe (10/10, 20/10, 40/10 or 80/10 mg daily), lovastatin (10, 20, 40 or 80 mg daily), pravastatin (10, 20, 40 or 80 mg daily), and combinations thereof. Other lipid lowering therapies that an ANGPTL3 inhibitor may be administered in combination with in the context of the present invention include, *e.g*., (1) an agent which inhibits cholesterol uptake and or bile acid re-absorption (*e.g*., ezetimibe); (2) an agent which increase lipoprotein catabolism (such as niacin); and/or (3) activators of the LXR transcription factor that plays a role in cholesterol elimination such as 22-hydroxycholesterol.

According to certain embodiments, the present invention comprises administering an ANGPTL3 inhibitor to a subject in combination with an inhibitor of ANGPTL4 (*e.g*., an anti-ANGPTL4 antibody or antigen-binding fragment thereof), an inhibitor of ANGPTL8 (*e.g.*, an anti-ANGPTL8 antibody or antigen-binding fragment thereof), and/or an inhibitor of PCSK9 *(e.g.,* an anti-PCSK9 antibody or antigen-binding fragment thereof). Non-limiting examples of anti-PCSK9 antibodies that can be used in the context of the present invention include, *e.g*., alirocumab, evolocumab, bococizumab, lodelcizumab, ralpancizumab, or antigen-binding portions of any of the foregoing antibodies.

### Administration Regimens

According to certain embodiments of the present invention, multiple doses of an ANGPTL3 inhibitor (*i.e*., a pharmaceutical composition comprising an ANGPTL3 inhibitor) may be administered to a subject over a defined time course (*e.g*., on top of a daily therapeutic statin regimen or other background lipid lowering therapy). The methods according to this aspect of the invention comprise sequentially administering to a subject multiple doses of an ANGPTL3 inhibitor. As used herein, "sequentially administering" means that each dose of an ANGPTL3 inhibitor is administered to the subject at a different point in time, *e.g*., on different days separated by a predetermined interval (*e.g*., hours, days, weeks or months). The present invention includes sequentially administering to the patient a single initial dose of an ANGPTL3 inhibitor, followed by one or more secondary doses of the ANGPTL3 inhibitor, and optionally followed by one or more tertiary doses of the ANGPTL3 inhibitor.

The terms "initial dose," "secondary doses," and "tertiary doses," refer to the temporal sequence of administration of the individual doses of a pharmaceutical composition comprising an ANGPTL3 inhibitor. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of the ANGPTL3 inhibitor, but generally may differ from one another in terms of frequency of administration. In certain embodiments, however, the amount of ANGPTL3 inhibitor contained in the initial, secondary and/or tertiary doses varies from one another (*e.g*., adjusted up or down as appropriate) during the course of treatment. In certain embodiments, two or more (*e.g.,* 2, 3, 4, or 5) doses are administered at the beginning of the treatment regimen as "loading doses" followed by subsequent doses that are administered on a less frequent basis (*e.g*., "maintenance doses").

According to exemplary embodiments of the present invention, each secondary and/or tertiary dose is administered 1 to 26 (*e.g.,* 1, 1½, 2, 2½, 3, 3½, 4, 4½, 5, 5½, 6, 6½, 7, 7½, 8, 8½, 9, 9½, 10, 10½, 11, 11½, 12, 12½, 13, 13½, 14, 14½, 15, 15½, 16, 16½, 17, 17½, 18, 18½, 19, 19½, 20, 20½, 21, 21½, 22, 22½, 23, 23½, 24, 24½, 25, 25½, 26, 26½, or more) weeks after the immediately preceding dose. The phrase "the immediately preceding dose," as used herein, means, in a sequence of multiple administrations, the dose of antigen-binding molecule which is administered to a patient prior to the administration of the very next dose in the sequence with no intervening doses.

The methods according to this aspect of the invention may comprise administering to a patient any number of secondary and/or tertiary doses of an ANGPTL3 inhibitor. For example, in certain embodiments, only a single secondary dose is administered to the patient. In other embodiments, two or more (*e.g.,* 2, 3, 4, 5, 6, 7, 8, or more) secondary doses are administered to the patient. Likewise, in certain embodiments, only a single tertiary dose is administered to the patient. In other embodiments, two or more (*e.g.,* 2, 3, 4, 5, 6, 7, 8, or more) tertiary doses are administered to the patient.

In embodiments involving multiple secondary doses, each secondary dose may be administered at the same frequency as the other secondary doses. For example, each secondary dose may be administered to the patient 1 to 2, 4, 6, 8 or more weeks after the immediately preceding dose. Similarly, in embodiments involving multiple tertiary doses, each tertiary dose may be administered at the same frequency as the other tertiary doses. For example, each tertiary dose may be administered to the patient 1 to 2, 4, 6, 8 or more weeks after the immediately preceding dose. Alternatively, the frequency at which the secondary and/or tertiary doses are administered to a patient can vary over the course of the treatment regimen. The frequency of administration may also be adjusted during the course of treatment by a physician depending on the needs of the individual patient following clinical examination.

### EXAMPLES

The following examples are put forth so as to provide those of ordinary skill in the art with a complete disclosure and description of how to use an angiopoietin-like protein-3 (ANGPTL3) inhibitory antibody as defined in the claims to reduce the progression of atherosclerosis. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. Generation of Human Antibodies to Human ANGPTL3

Human anti-ANGPTL3 antibodies were generated as described in US Patent No. 9,018,356. The exemplary ANGPTL3 inhibitor used in the following Example is the human anti-ANGPTL3 antibody designated "H4H1276S," also known as "evinacumab." Evinacumab has the following amino acid sequence characteristics: a heavy chain comprising SEQ ID NO:10 and a light chain comprising SEQ ID NO:11; a heavy chain variable region (HCVR) comprising SEQ ID NO:2 and a light chain variable domain (LCVR) comprising SEQ ID NO:3; a heavy chain complementarity determining region 1 (HCDR1) comprising SEQ ID NO:4, a HCDR2 comprising SEQ ID NO:5, a HCDR3 comprising SEQ ID NO:6, a light chain complementarity determining region 1 (LCDR1) comprising SEQ ID NO:7, a LCDR2 comprising SEQ ID NO:8 and a LCDR3 comprising SEQ ID NO:9.

### Example 2. An Anti-ANGPTL3 Antibody Prevents Atherosclerosis Development in a Mouse Model

### INTRODUCTION

The objective of this study was to evaluate the effect of ANGPTL3 inhibition on the development of atherosclerosis in APOE*3Leiden.CETP mice, a well-established model for hyperlipidemia with all features of mixed or Familial Dysbetalipoproteinemia, and atherosclerosis. (*See, e.g.,* Kühnast et al., 2014, J Lipid Res 55: 2103-2112, for a general description of the APOE*3Leiden.CETP mouse model (alternatively referred to herein as "E3L.CETP mice") and its use in assessing the effects of a pharmacologic agent on atherosclerosis development).

ANGPTL3 inhibition was achieved using an anti-ANGPTL3 antibody. The particular anti-ANGPTL3 antibody used in these studies was the antibody referred to as evinacumab as described above. The control antibody used in these studies is an isotype-matched antibody to an irrelevant target.

### STUDY DESIGN

An illustration of the study design is shown in Figure 1. Evinacumab is a fully human IgG4 antibody. Thus, when evaluated in different mouse strains, some mice develop anti-drug antibodies that eliminate the efficacy of the therapeutic. The percentage of mice developing this anti-drug response can be different for each mouse model/genetic background. For this reason more mice per group were included in this first study in APOE*3Leiden.CETP mice to determine how many mice develop an anti-human immune response in this particular model. After exclusion of the mice developing an anti-human immune response the remaining mice could be used for evaluating the effect of evinacumab on atherosclerosis.

Sixty-three female APOE*3Leiden.CETP mice were put on diet T (a semi-synthetic modified diet as described by Nishina et al. 1990, J Lipid Res 31:859, with 0.15% added cholesterol and 15% cocoa butter. After a 4 weeks run-in period 13 low-responder mice were removed from the study and the remaining 50 mice were sub-divided into one control group of twenty mice and one evinacumab treatment group of thirty mice, matched for body weight, plasma cholesterol and triglycerides after 4h fasting (t=0).

Treatment with evinacumab or control antibody was given weekly via subcutaneous injections at a dose of 25 mg/kg/wk. The injection volume during the treatment period was 10 mUkg using 0.9%NaCl as vehicle and was adjusted to the lastly measured body weight value. Body weight and food intake (per cage) were measured at week 0, 3, 6, 8, 11 and 13.

After 0, 2, 4, 6, 8, 11 and 13 weeks of treatment blood samples were taken after four hours of fasting. Plasma cholesterol and triglycerides were measured individually and additional EDTA plasma was collected for hFc and mouse anti-human antibody evaluation. After 6 weeks of treatment the first anti-drug response was evaluated and 3 mice of the control group and 5 mice of the treatment group with high levels of anti-drug antibodies were removed from the study.

Lipoprotein profiles were measured at week 0, 6 and 13 in plasma samples, pooled per group. Feces were collected per cage during week 12 (twice for 48 hours). At t=13 mice were sacrificed non-fasted. EDTA-plasma was obtained via heart puncture and several tissues were collected. Atherosclerosis development in the aortic root was measured in 15 mice per group (after exclusion of the mice that developed an anti-drug response) by measuring lesion number, lesion area and lesion severity. In addition, lesion composition was determined.
(1) The schedule of parameter measurements is as follows:
(2) Body weight (week 0, 3, 6, 8, 11 and 13);
(3) Food intake (per cage, week 0, 3, 6, 8, 11 and 13);
(4) Plasma total cholesterol and triglycerides (after 4h fasting at week 0, 2, 4, 6, 8, 11 and 13);
(5) Collection of 20 µl EDTA plasma for hFc and mouse anti-human antibody evaluation (after 4h fasting at week 0, 2, 4, 6, 8, 11 and 13);
(6) Lipoprotein profiles (cholesterol and triglycerides, pooled per group; at week 0, 6 and 13);
(7) Atherosclerosis development in the aortic root (15 mice per group): (a) Lesion number, (b) Total lesion area, (c) Lesion severity, (d) Lesion composition (e.g. macrophage, smooth muscle cell in the cap, necrosis and collagen content), and (e) Monocyte adherence. (At sacrifice [week 13], aortas were collected from all mice, however atherosclerosis analysis was performed with 15 mice per group; mice that did not develop anti-drug antibodies were selected).

### METHODS

Body weight and food intake measurements were performed according to standard procedures.

Plasma blood samples were obtained according to standard procedures. Total plasma cholesterol and triglycerides were determined using commercially available kits.

Measurement of lipoprotein profiles by FPLC analysis using an AKTA apparatus from Amersham Biosciences. Analyses were performed in samples pooled per group. Cholesterol and triglycerides were measured in the fractions using commercially available kits.

Atherosclerotic lesion area and severity was assessed in the aortic root area as reported previously (see, e.g., Kühnast et al., 2012, J Hypertens 30:107-116). The aortic root was identified by the appearance of aortic valve leaflets and serial cross-sections of the entire aortic root area (5 µm thick with intervals of 50 µm) were mounted on 3-aminopropyl triethoxysilane-coated slides and stained with hematoxylin-phloxine-saffron (HPS). For each mouse, the lesion area was measured in 4 subsequent sections. Each section consists of 3 segments (separated by the valves). For determination of atherosclerotic lesion size and severity, the lesions were classified into five categories according to the American Heart Association (AHA) (see Stary et al., 1995, Arterioscler Thromb Vasc Biol 15:1512-1531): Type 1: Early fatty streaks (up to 10 foam cells in the intima with no other changes); Type 2: Regular fatty streaks (ten or more foam cells in the intima with no other changes); Type 3: Mild plaque (foam cells with a fibrotic cap); Type 4: Moderate plaque (more progressed lesions with an affected media, but without loss of architecture of the media); Type 5: Severe plaque (the media is severely affected; broken elastic fibers, cholesterol clefts, calcification and necrosis are frequently observed).

Images were taken with an Olympus BX51 microscope and areas were measured with Image Processing software. The total lesion area and number of lesions were calculated per cross-section. Undiseased segments were calculated as percentage of total segments. Lesion severity as percentage of total lesions was also determined. Type I-III lesions were classified as mild lesions and type IV-V lesions were classified as severe lesions.

The number of monocytes adhering to the activated endothelium was counted in each segment used for lesion quantification, after immunostaining with AIA 31240 antiserum (1:1000; Accurate Chemical and Scientific, New York, New York, USA) and calculated per cross section.

Macrophage area of the lesions was measured after immunostaining with anti-mouse Mac-3 (1:50; BD Pharmingen, the Netherlands). Sirius Red staining was performed to stain collagen. Smooth muscle cell area of the fibrotic cap was measured after immunostaining with anti-alpha smooth muscle actin (1:400; PROGEN Biotechnik GmbH, Heidelberg, Germany) which cross-reacts with mouse alpha actin. Photos/Images of the lesions were taken with the Olympus BX51 microscope. Macrophage, collagen, necrotic core and smooth muscle cell area of the lesions were quantified using imaging software. The macrophage, smooth muscle cells (SMCs), necrotic core and collagen content of the lesions were calculated as percentage of the lesion area. Plaque vulnerability/stability index was calculated by dividing the sum of stabilizing factors SMCs and collagen by the sum of destabilization factors, macrophages and necrotic core.

At week 13 mice were sacrificed non-fasted via CO2. No last antibody injection was given during week 13 before sacrifice. After sacrifice, as much EDTA-plasma as possible was collected (via heart puncture) and stored at -70°C until further use. Hearts including aortic root were fixed in 10% formalin. Thoracic aorta was collected and fixed in 10% formalin.

### STATISTICS

Depending on normality, significance of differences between the groups were calculated non-parametrically, using the computer program SPSS and a Mann-Whitney U-test for independent samples. A P-value<0.05 was considered statistically significant. Statistical differences of the treatment group as compared to the control group are indicated with an asterisk at the measure points in the figures

### RESULTS

### BODY WEIGHT

Body weight results are summarized in Table 1. Values are absolute values (grams) and are means ± S.D. from 14-15 mice per group.

**TABLE 1**

| **Body weight (g)** | | | | | | | |
|---|---|---|---|---|---|---|---|
| | Time (weeks) | t=0 | t=3 | t=6 | t=8 | t=11 | t=13 |
| Control antibody (AB) | AVG | 21.8 | 21.6 | 22.1 | 22.7 | 23.6 | 23.4 |
| | SD | 1.4 | 1.2 | 1.3 | 1.4 | 1.7 | 2.0 |
| Evinacumab | AVG | 21.3 | 21.6 | 21.8 | 22.2 | 23.1 | 23.0 |
| | SD | 1.4 | 1.5 | 1.4 | 1.3 | 1.5 | 1.7 |
| | | | | | | | |
| Body weight: P-value | | t=0 | t=3 | t=6 | t=8 | t=11 | t=13 |
| Control vs. evinacumab | | 0.400 | 0.747 | 0.425 | 0.477 | 0.715 | 0.847 |

| | | | | | | | |
|---|---|---|---|---|---|---|---|
| Non parametric analysis: Mann Whitney U test | | | | | | | |

There were no differences in body weight between the evinacumab-treated group as compared to the control group.

### FOOD INTAKE

Food intake results are summarized in Table 2. Values are absolute values (gram/mouse/day) and are means ± S.D. of 17 cages (all mice; before matching) or 4-6 cages (after matching and start treatment).

**TABLE 2**

| **Food Intake (g/m/d)** | | | | | | | |
|---|---|---|---|---|---|---|---|
| | Time (weeks) | t=0 | t=3 | t=6 | t=8 | t=11 | t=13 |
| Control AB | AVG | 2.9 | 2.6 | 2.4 | 2.3 | 2.6 | 2.5 |
| | SD | 0.2 | 0.1 | 0.2 | 0.2 | 0.4 | 0.4 |
| Evinacumab | AVG | 2.9 | 2.7 | 2.4 | 2.3 | 2.5 | 2.5 |
| | SD | 0.2 | 0.1 | 0.2 | 0.2 | 0.3 | 0.4 |
| | | | | | | | |
| Food intake: P-value | | t=0 | t=3 | t=6 | t=8 | t=11 | t=13 |
| Control vs. evinacumab | | n/a | 0.476 | 0.914 | 0.914 | 0.914 | 1.000 |

| | | | | | | | |
|---|---|---|---|---|---|---|---|
| Non parametric analysis: Mann Whitney U test | | | | | | | |

There were no differences in food intake for the evinacumab-treated group as compared to the control group.

### PLASMA CHOLESTEROL

Plasma cholesterol results are summarized in Tables 3 and 4, and Figures 2A and 2B. Values are absolute values (mM) and are means ± S.D. from 14-15 mice per group.

**TABLE 3**

| **Cholesterol (mM)** | | | | | | | | |
|---|---|---|---|---|---|---|---|---|
| | Time (weeks) | t=0 | t=2 | t=4 | t=6 | t=8 | t=11 | t=13 |
| Control AB | AVG | 24.1 | 29.9 | 28.0 | 26.7 | 27.6 | 22.1 | 20.9 |
| | SD | 3.1 | 5.0 | 4.5 | 3.3 | 4.5 | 5.4 | 4.6 |
| Evinacumab | AVG | 23.7 | 12.6 | 14.0 | 14.1 | 13.6 | 10.8 | 9.6 |
| | SD | 2.4 | 2.4 | 1.9 | 2.2 | 2.5 | 1.7 | 2.1 |
| | | | | | | | | |
| Cholesterol: | | t=0 | t=2 | t=4 | t=6 | t=8 | t=11 | t=13 |
| P-value | | | | | | | | |
| Control vs. evinacumab | | 0.9.14 | <0.001 | <0.001 | <0.001 | <0.001 | <0.001 | <0.001 |

| | | | | | | | | |
|---|---|---|---|---|---|---|---|---|
| Non parametric analysis: Mann Whitney U test | | | | | | | | |

**TABLE 4**

| **Cholesterol (mM)** | | Total cholesterol exposure (mM*wks) |
|---|---|---|
| | Time (weeks) | t=13 |
| Control AB | AVG | 393.5 |
| | SD | 49.7 |
| Evinacumab | AVG | 230.2 |
| | SD | 23.8 |
| | | |
| Cholesterol exposure: P-value | | t=13 |
| Control vs. evinacumab | | <0.001 |

| | | |
|---|---|---|
| Non parametric analysis: Mann Whitney U test | | |

Treatment with evinacumab, led to a significant decrease in plasma cholesterol as compared to the control group at all time-points (average decrease of 52%). At the end of the experiment the cholesterol exposure during the experiment (0-13 weeks + run-in period) was significantly decreased upon treatment with evinacumab (with 41%, p<0.001) as compared to the control group.

### PLASMA TRIGLYCERIDES

Plasma triglycerides results are summarized in Table 5, and Figure 3. Values are absolute values (mM) and are means ± S.D. from 14-15 mice per group.

**TABLE 5**

| **Triglycerides (mM)** | | | | | | | | |
|---|---|---|---|---|---|---|---|---|
| | Time (weeks) | t=0 | t=2 | t=4 | t=6 | t=8 | t=11 | t=13 |
| Control AB | AVG | 7.5 | 5.5 | 4.9 | 4.8 | 3.6 | 4.0 | 3.4 |
| | SD | 2.6 | 1.3 | 1.6 | 1.5 | 1.3 | 1.0 | 1.2 |
| Evinacumab | AVG | 7.4 | 0.7 | 0.7 | 0.7 | 0.7 | 0.7 | 0.6 |
| | SD | 2.0 | 0.3 | 0.2 | 0.3 | 0.2 | 0.2 | 0.2 |
| | | | | | | | | |
| Triglycerides: | | t=0 | t=2 | t=4 | t=6 | t=8 | t=11 | t=13 |
| P-value | | | | | | | | |
| Control vs. evinacumab | | 0.747 | <0.001 | <0.001 | <0.001 | <0.001 | <0.001 | <0.001 |

| | | | | | | | | |
|---|---|---|---|---|---|---|---|---|
| Non parametric analysis: Mann Whitney U test | | | | | | | | |

Treatment with evinacumab, led to a significant decrease in plasma triglycerides as compared to the control group at all time-points (average decrease of 84%).

### LIPOPROTEIN PROFILES

Lipoproteins were separated on a Superose column. Cholesterol and triglyceride fractions at t=0, t=6 weeks, and t=13 weeks, are shown in Figures 4A, 4B, and 4C [cholesterol] and 5A, 5B, and 5C [triglycerides]. Values are absolute values from cholesterol and triglyceride measurements in pooled plasma per group at t=0, 6 and 13 weeks. For purposes of this analysis, fractions 3-7 were considered as VLDL; 8-16 as IDL/LDL and 17-24 as HDL.

At t=0, before treatments were started, no differences in lipoprotein profiles were observed, while after 6 and 13 weeks of treatment with evinacumab the plasma cholesterol levels, measured in the samples pooled per group, were decreased in the VLDL-LDL peak as compared to the control group, and appeared to be increased in the HDL peak (see Figures 4B and 4C). Triglyceride profiles showed a similar pattern (see Figures 5A, 5B, and 5C).

These data are in line with the observed decreases in individual animal total cholesterol and triglyceride levels during the study.

### ATHEROSCLEROSIS LESION AREA

Atherosclerotic lesion area is summarized in Table 6, and Figures 6A and 6B. Values are absolute lesion areas in the aortic root per cross section and expressed as means ± S.D. of 14-15 mice per group.

**TABLE 6**

| **Lesion area per cross section (*1000 µm2)** | | |
|---|---|---|
| | Time (weeks) | t=13 |
| Control AB | AVG | 216.1 |
| | SD | 49.3 |
| Evinacumab | AVG | 133.0 |
| | SD | 52.8 |
| | | |
| Lesion area: P-value | | t=13 |
| Control vs. evinacumab | | 0.001 |

| | | |
|---|---|---|
| Non parametric analysis: Mann Whitney U test | | |

The total lesion area in the aortic root of the control group was 216082 µm² per cross section at sacrifice at t=13 weeks. Treatment with evinacumab significantly decreased the total lesion area with 39% (p<0.001) as compared to the control group at sacrifice.

### ATHEROSCLEROSIS: NUMBER OF LESIONS

The number of atherosclerotic lesions per cross-section is summarized in Table 7. Values are absolute values (number of lesions) and expressed as means ± S.D. of 14-15 mice per group.

**TABLE 7**

| **Number of lesions per cross section (*1000 µm2)** | | |
|---|---|---|
| | Time (weeks) | t=13 |
| Control AB | AVG | 5.0 |
| | SD | 0.8 |
| Evinacumab | AVG | 5.0 |
| | SD | 1.1 |
| | | |
| Number of lesions: P-value | | t=13 |
| Control vs. evinacumab | | 0.880 |

| | | |
|---|---|---|
| Non parametric analysis: Mann Whitney U test | | |

There were no differences in the number of lesions between the evinacumab-treated group as compared to the control group.

### ATHEROSCLEROSIS: LESION SEVERITY

Atherosclerotic lesion severity results are summarized in Table 8. Values are percentages of total lesions and expressed as means ± S.D. of 14-15 mice per group.

**TABLE 8**

| **Lesion severity (%)** | | | |
|---|---|---|---|
| | Time (weeks) | Mild lesions (Type I-III) | Severe lesions (Type IV-V) |
| Control AB | AVG | 27.7 | 72.3 |
| | SD | 10.6 | 10.6 |
| Evinacumab | AVG | 36.8 | 63.2 |
| | SD | 26.8 | 26.8 |
| | | | |
| Lesion severity: P-value | | Mild (I-III) | Severe (IV-V) |
| Control vs. evinacumab | | 0.451 | 0.451 |

| | | | |
|---|---|---|---|
| Non parametric analysis: Mann Whitney U test | | | |

There were no differences in lesion severity between the evinacumab-treated group as compared to the control group.

### ATHEROSCLEROSIS: UNDISEASED SEGMENTS

The number of undiseased atherosclerotic segments is summarized in Table 9. Values represent the percentage of non-atherosclerotic ("undiseased" or healthy) segments. The values are means ± S.D. of 14-15 mice per group.

**TABLE 9**

| **Undiseased segments (%)** | | |
|---|---|---|
| | Time (weeks) | t=13 |
| Control AB | AVG | 2.4 |
| | SD | 8.9 |
| Evinacumab | AVG | 2.8 |
| | SD | 8.7 |
| | | |
| Undiseased segments: P-value | | t=13 |
| Control vs. evinacumab | | 0.813 |

| | | |
|---|---|---|
| Non parametric analysis: Mann Whitney U test | | |

There were no differences in the amount of undiseased segments between the evinacumab-treated group as compared to the control group.

### ATHEROSCLEROSIS: MACROPHAGE CONTENT

The macrophage content of the lesions is summarized in Table 10 and Figure 7A. Values are absolute values (µm² *1000) or relative values (% of lesion) and expressed as means ± S.D. of 14-15 mice per group.

**TABLE 10**

| | | Macrophage area / cross section (µm2*1000) | | Macrophage content (% of lesion) | |
|---|---|---|---|---|---|
| | | Type III-V | Type IV-V | Type III-V | Type IV-V |
| Control AB | AVG | 25.9 | 22.7 | 12.9 | 12.1 |
| | SD | 8.3 | 8.3 | 4.7 | 4.9 |
| Evinacumab | AVG | 19.3 | 15.9 | 17.2 | 14.6 |
| | SD | 9.7 | 10.6 | 7.7 | 5.6 |
| | | | | | |

| Macrophage area: P-value | | Macrophage area / cross section (µm2*1000) | | Macrophage content (% of lesion) | |
|---|---|---|---|---|---|
| | | Type III-V | Type IV-V | Type III-V | Type IV-V |
| Control vs. evinacumab | | 0.070 | 0.070 | 0.102 | 0.227 |

| | | | | | |
|---|---|---|---|---|---|
| Non parametric analysis: Mann Whitney U test | | | | | |

Treatment with evinacumab tended to decrease the macrophage content per cross section in the atherosclerotic lesions type IV-V as compared to the control group. This difference was not observed, however, after correction for total lesion area and expressed as percentage of macrophages in the type IV-V lesions (Figure 7A).

### ATHEROSCLEROSIS: NECROTIC CONTENT

The necrotic content of the lesions is summarized in Table 11 and Figure 7A. Values are absolute values (µm²*1000) or relative values (% of lesion) and expressed as means ± S.D. of 14-15 mice per group.

**TABLE 11**

| | | Necrotic area / cross section (µm2*1000) | | Necrotic content (% of lesion) | |
|---|---|---|---|---|---|
| | | Type III-V | Type IV-V | Type III-V | Type IV-V |
| Control AB | AVG | 19.4 | 18.8 | 8.8 | 9.0 |
| | SD | 10.2 | 10.2 | 3.4 | 3.6 |
| Evinacumab | AVG | 6.0 | 5.8 | 4.5 | 4.9 |
| | SD | 3.8 | 3.9 | 2.0 | 1.8 |
| | | | | | |

| Necrotic area: P-value | | Necrotic area / cross section (µm2*1000) | | Necrotic content (% of lesion) | |
|---|---|---|---|---|---|
| | | Type III-V | Type IV-V | Type III-V | Type IV-V |
| Control vs. evinacumab | | **<0.001** | **<0.001** | **<0.001** | **0.001** |

| | | | | | |
|---|---|---|---|---|---|
| Non parametric analysis: Mann Whitney U test | | | | | |

Treatment with evinacumab significantly decreased the necrotic content per cross section in the atherosclerotic lesions type IV-V as compared to the control group (with 69%, p<0.001). After correction for total lesion area the percentage of the necrotic area in the type IV-V lesions was also significantly decreased as compared to the control group (with 45%, p=0.001) (Figure 7A).

### ATHEROSCLEROSIS: SMOOTH MUSCLE CELLS IN FIBROTIC CAP

The quantification of smooth muscle cells (SMCs) in the fibrotic cap of the lesions is summarized in Table 12 and Figure 7B. Values are absolute values (µm²*1000) or relative values (% of lesion) and expressed as means ± S.D. of 14-15 mice per group.

**TABLE 12**

| | | SMC area / cross section (µm2*1000) | | SMC (% of lesion) | |
|---|---|---|---|---|---|
| | | Type III-V | Type IV-V | Type III-V | Type IV-V |
| Control AB | AVG | 24.9 | 23.4 | 12.1 | 12.1 |
| | SD | 7.7 | 7.7 | 3.2 | 3.3 |
| Evinacumab | AVG | 15.8 | 14.4 | 13.5 | 13.7 |
| | SD | 7.5 | 8.0 | 4.2 | 4.6 |
| | | | | | |

| SMC area: P-value | | SMC area / cross section (µm2*1000) | | SMC content (% of lesion) | |
|---|---|---|---|---|---|
| | | Type III-V | Type IV-V | Type III-V | Type IV-V |
| Control vs. evinacumab | | **0.005** | **0.009** | 0.217 | 0.210 |

| | | | | | |
|---|---|---|---|---|---|
| Non parametric analysis: Mann Whitney U test | | | | | |

Treatment with evinacumab significantly decreased the smooth muscle cell (SMC) area per cross section in the atherosclerotic lesions type IV-V as compared to the control group (with 39%, p=0.009). However, after correction for total lesion, the percentage of SMC in the fibrotic cap in the type IV-V lesions was not significantly decreased as compared to the control group (Figure 7B).

### ATHEROSCLEROSIS: COLLAGEN CONTENT

The collagen content of the lesions is summarized in Table 13 and Figure 7B. Values are absolute values (µm²*1000) or relative values (% of lesion) and expressed as means ± S.D. of 14-15 mice per group.

**TABLE 13**

| | | Collagen area/cross section (µm2*1000) | | Collagen content (% of lesion) | |
|---|---|---|---|---|---|
| | | Type III-V | Type IV-V | Type III-V | Type IV-V |
| Control AB | AVG | 102.6 | 96.9 | 48.7 | 48.5 |
| | SD | 34.0 | 34.7 | 8.7 | 9.2 |
| Evinacumab | AVG | 62.0 | 58.1 | 48.7 | 52.3 |
| | SD | 29.2 | 30.5 | 10.3 | 8.2 |
| | | | | | |

| Collagen area: P-value | | Collagen area / cross section (µm2*1000) | | Collagen content (% of lesion) | |
|---|---|---|---|---|---|
| | | Type III-V | Type IV-V | Type III-V | Type IV-V |
| Control vs. evinacumab | | **0.005** | **0.009** | 0.813 | 0.227 |

| | | | | | |
|---|---|---|---|---|---|
| Non parametric analysis: Mann Whitney U test | | | | | |

Treatment with evinacumab significantly decreased the collagen content per cross section in the atherosclerotic lesions type IV-V as compared to the control group (with 40%, p=0.009). However, after correction for total lesion area, the percentage of collagen in the type IV-V lesions was not significantly decreased as compared to the control group (Figure 7B).

### ATHEROSCLEROSIS: MONOCYTE ADHESION

The percent of monocytes per cross-section is summarized in Table 14. Values are absolute values (number of adherent monocytes per cross section) and expressed as means ± S.D. of 14-15 mice per group.

**TABLE 14**

| | | Number of monocytes per cross section |
|---|---|---|
| Control AB | AVG | 3.4 |
| | SD | 1.2 |
| Evinacumab | AVG | 3.5 |
| | SD | 1.2 |
| | | |

| Monocytes: P-value | | Number of monocytes per cross section |
|---|---|---|
| Control vs. evinacumab | | 0.983 |

| | | |
|---|---|---|
| Non parametric analysis: Mann Whitney U test | | |

Monocyte adhesion to the activated endothelium in the vessel wall of the aortic root was not affected by treatment with evinacumab as compared to the control group.

### ATHEROSCLEROSIS: STABILITY INDEX

Plaque stability of the lesions (expressed in terms of a stability index) is summarized in Table 15. The stability index is defined as the sum of the stabilization markers SMC and collagen content divided by the sum of the destabilization markers macrophage and necrotic content. This value provides a measure for the plaque stability. The values are means ± S.D. of 14-15 mice per group.

**TABLE 15**

| | | Stability index in type IV-V |
|---|---|---|
| | | (% SMC + % collagen) / (% macrophage + % necrosis) |
| Control AB | AVG | 3.2 |
| | SD | 1.3 |
| Evinacumab | AVG | 3.7 |
| | SD | 1.7 |
| | | |

| Stability index: P-value | | Stability index in type IV-V |
|---|---|---|
| | | (% SMC + % collagen) / (% macrophage + % necrosis) |
| Control vs. evinacumab | | 0.329 |

| | | |
|---|---|---|
| Non parametric analysis: Mann Whitney U test | | |

Treatment with evinacumab did not have an effect on the stability index as compared to the control group.

### SUMMARY

### SAFETY ASPECTS

No effects on body weight (gain) and food intake were noted by evinacumab treatment as compared to control.

### REDUCTION IN VLDL AND LDL LEVELS

The APOE*3Leiden.CETP mice are a well-established model for familial dysbetalipoproteinemia (FD) in humans which is characterized by accumulation of remnant lipoproteins and an increased VLDL-C to LDL-C ratio. The lipoprotein profile in APOE*3Leiden.CETP mice reflects that of FD patients with a similar response to lipid modifying therapies. This is illustrated by a comparable reduction in cholesterol in all apoB-containing lipoprotein subfractions with statin treatment 25 and fenofibrate and niacin.

In the present study, treatment with evinacumab significantly decreased average total cholesterol (TC) (-52%, p<0.001) and triglycerides (TG) (-84%, p<0.001) as compared to control, which was confined to non-HDL fractions.

### REDUCTION IN LESION SIZE AND NECROTIC AREA

In the current study, evinacumab significantly decreased lesion size (-39%, p<0.001) as compared to the control group. No significant differences were found in the number of lesions, undiseased segments or lesion severity.

Treatment with evinacumab significantly decreased necrotic content as percentage of the total lesion size in severe type IV-V lesions (with 45%, p=0.001), but did not affect macrophage content, collagen content or SMC area, resulting in a similar lesion stability index as the control group. Evinacumab did not affect the monocyte recruitment to the activated endothelium.

The necrotic content was the only marker of plaque composition that was changed in terms of both absolute area and also as a percentage of total lesion area. A potential explanation may be a shift in the composition of the macrophage population in the plaque from the more pro-inflammatory M1 "killer" macrophages to the more healing "repair" M2 macrophages, which may remove cholesterol from the plaque.

### CONCLUSION

Collectively, the results set forth in this Example show that the anti-ANGPTL3 monoclonal antibody, evinacumab, decreases plasma lipids and prevents progression of atherosclerosis in APOE*3Leiden.CETP mice. Evinacumab did not have an effect on the number of lesions, lesion severity or plaque stability, but significantly reduced lesion area and the necrotic core area.

## Claims

1. An angiopoietin-like protein 3 (ANGPTL3) inhibitor for use in a method of reducing the progression of atherosclerosis in a subject, the method comprising selecting a subject who has atherosclerosis and administering one or more doses of the angiopoietin-like protein 3 inhibitor to the subject, wherein the ANGPTL3 inhibitor is an antibody or antigen-binding fragment thereof that specifically binds ANGPTL3 which comprises heavy and light chain CDR amino acid sequences having SEQ ID NOs:4, 5, 6, 7, 8, and 9.

2. The ANGPTL3 inhibitor for use in the method of claim 1, wherein the subject, prior to or at the time of initiation of treatment with the one or more doses of the ANGPTL3 inhibitor, is diagnosed with heterozygous familial hypercholesterolemia (HeFH) or homozygous familial hypercholesterolemia (HoFH), and/or elevated lipoprotein(a) (Lp[a]).

3. The ANGPTL3 inhibitor for use in the method of claim 1, wherein the subject, prior to or at the time of initiation of treatment with the one or more doses of the ANGPTL3 inhibitor, is diagnosed with cardiovascular disease (CVD), or has suffered a stroke or myocardial infarction.

4. The ANGPTL3 inhibitor for use in the method of any one of claims 1 to 3, wherein the subject is on a stable background lipid modifying therapy (LMT) prior to or at the time of initiation of treatment with the one or more doses of the ANGPTL3 inhibitor, or concurrent with administration of the one or more doses of the ANGPTL3 inhibitor, optionally wherein the stable background LMT is low-, moderate-, or high-dose statin therapy.

5. The ANGPTL3 inhibitor for use in the method of any one of claims 1 to 4, wherein administration of the one or more doses of the ANGPTL3 inhibitor to the subject results in one or more therapeutic consequence(s) selected from the group consisting of:
(a) a reduction in serum total cholesterol (TC) level,
(b) a reduction in serum triglyceride (TG) level,
(c) a reduction in serum low-density lipoprotein (LDL) level, and
(d) a reduction in serum very low density lipoprotein (VLDL) level;
wherein the reduction of (a), (b), (c) and/or (d) is determined relative to the subject's serum TC level, serum TG level, serum LDL level, and/or serum VLDL level prior to or at the time of initiation of treatment with the one or more doses of the ANGPTL3 inhibitor.

6. The ANGPTL3 inhibitor for use in the method of any one of claims 1 to 5, wherein administration of the one or more doses of the ANGPTL3 inhibitor to the subject results in a reduction in atherosclerotic plaque formation.

7. The ANGPTL3 inhibitor for use in the method of any one of the preceding claims, wherein the antibody or antigen-binding fragment thereof that specifically binds ANGPTL3 comprises an HCVR having the amino acid sequence of SEQ ID NO:2 and an LCVR having the amino acid sequence of SEQ ID NO:3.

8. The ANGPTL3 inhibitor for use in the method of claim 1, wherein the antibody or antigen-binding fragment thereof that specifically binds ANGPTL3 is evinacumab.

9. The ANGPTL3 inhibitor for use in the method of any one of claims 1 to 8, further comprising administering one or more doses of a proprotein convertase subtilisin kexin-9 (PCSK9) inhibitor to the subject, wherein the PCSK9 inhibitor is an antibody or antigen-binding fragment thereof that specifically binds PCSK9.

10. The ANGPTL3 inhibitor for use in the method of claim 9, wherein the antibody or antigen-binding fragment thereof that specifically binds PCSK9 is selected from the group consisting of alirocumab, evolocumab, bococizumab, lodelcizumab, and ralpancizumab.

11. The ANGPTL3 inhibitor for use in the method of any one of claims 1 to 10, further comprising administering one or more doses of an ANGPTL4 inhibitor and/or ANGPTL8 inhibitor to the subject, wherein the ANGPTL4 inhibitor is an antibody or antigen-binding fragment thereof that specifically binds ANGPTL4, wherein the ANGPTL8 inhibitor is an antibody or antigen-binding fragment thereof that specifically binds ANGPTL8.

12. A pharmaceutical composition for use in a method of reducing the progression of atherosclerosis in a subject, wherein the composition comprises an angiopoietin-like protein 3 (ANGPTL3) inhibitor, and wherein one or more doses of the angiopoietin-like protein 3 (ANGPTL3) inhibitor are administered to the subject, wherein the ANGPTL3 inhibitor is an antibody or antigen-binding fragment thereof that specifically binds ANGPTL3 which comprises heavy and light chain CDR amino acid sequences having SEQ ID NOs:4, 5, 6, 7, 8, and 9.

## Patentansprüche

1. Inhibitor des Angiopoietin-ähnlichen Proteins 3 (ANGPTL3) für die Verwendung in einem Verfahren zum Reduzieren des Fortschreitens der Atherosklerose in einem Subjekt, wobei das Verfahren das Auswählen eines Subjekts mit Atherosklerose und das Verabreichen einer oder mehrerer Dosen des Inhibitors des Angiopoietin-ähnlichen Proteins 3 an das Subjekt umfasst, wobei der ANGPTL3-Inhibitor ein Antikörper oder ein antigenbindendes Fragment davon ist, das spezifisch ANGPTL3 bindet, der CDR-Aminosäurensequenzen der schweren und leichten Kette mit den SEQ ID NO: 4, 5, 6, 7, 8 und 9 umfasst.

2. ANGPTL3-Inhibitor für die Verwendung in dem Verfahren nach Anspruch 1, wobei das Subjekt vor oder zu dem Zeitpunkt des Beginns der Behandlung mit der einen oder mehreren Dosen des ANGPTL3-Inhibitors mit heterozygoter familiärer Hypercholesterinämie (HeFH) oder homozygoter familiärer Hypercholesterinämie (HoFH) und/oder erhöhtem Lipoprotein(a) (Lp[a]) diagnostiziert wurde.

3. ANGPTL3-Inhibitor für die Verwendung in dem Verfahren nach Anspruch 1, wobei das Subjekt vor oder zu dem Zeitpunkt des Beginns der Behandlung mit der einen oder den mehreren Dosen des ANGPTL3-Inhibitors mit einer kardiovaskuläre Erkrankung (CVD) diagnostiziert wurde oder ein Schlaganfall oder ein Myokardinfarkt aufgetreten ist.

4. ANGPTL3-Inhibitor für die Verwendung in dem Verfahren nach einem der Ansprüche 1 bis 3, wobei das Subjekt vor oder zu dem Zeitpunkt des Beginns der Behandlung mit der einen oder den mehreren Dosen des ANGPTL3-Inhibitors oder gleichzeitig mit der Verabreichung der einen oder der mehreren Dosen des ANGPTL3-Inhibitors eine stabile lipidmodifizierende Hintergrundtherapie (LMT) erhält, optional wobei die stabile Hintergrund-LMT eine niedrig-, mittel- oder hochdosierte Statintherapie ist.

5. ANGPTL3-Inhibitor für die Verwendung in dem Verfahren nach einem der Ansprüche 1 bis 4, wobei die Verabreichung der einen oder mehreren Dosen des ANGPTL3-Inhibitors an das Subjekt zu einer oder mehrere therapeutische Folgen führt, ausgewählt aus der Gruppe, bestehend aus:
(a) einer Reduzierung des Gesamtcholesterinspiegels im Serum (TC),
(b) einer Reduzierung des Serumtriglyceridspiegels (TG),
(c) einer Reduzierung des Serumspiegels an Lipoproteinen niedriger Dichte (LDL), und
(d) einer Reduzierung des Serumspiegels an Lipoprotein sehr geringer Dichte (VLDL); wobei die Reduzierung von (a), (b), (c) und/oder (d) relativ zu dem
Serum-TC-Spiegel, Serum-TG-Spiegel, Serum-LDL-Spiegel und/oder Serum-VLDL-Spiegel des Subjekts vor oder zu dem Zeitpunkt des Beginns der Behandlung mit der einen oder den mehreren Dosen des ANGPTL3-Inhibitors bestimmt wird.

6. ANGPTL3-Inhibitor für die Verwendung in dem Verfahren nach einem der Ansprüche 1 bis 5, wobei die Verabreichung der einen oder der mehreren Dosen des ANGPTL3-Inhibitors an das Subjekt zu einer Reduktion der atherosklerotischen Plaquebildung führt.

7. ANGPTL3-Inhibitor für die Verwendung in dem Verfahren nach einem der vorhergehenden Ansprüche, wobei der Antikörper oder das antigenbindende Fragment davon, das spezifisch ANGPTL3 bindet, eine HCVR mit der Aminosäuresequenz von SEQ ID NO: 2 und eine LCVR mit der Aminosäuresequenz von SEQ ID NO: 3 umfasst.

8. ANGPTL3-Inhibitor für die Verwendung in dem Verfahren nach Anspruch 1, wobei der Antikörper oder das antigenbindende Fragment davon, das spezifisch ANGPTL3 bindet, Evinacumab ist.

9. ANGPTL3-Inhibitor für die Verwendung in dem Verfahren nach einem der Ansprüche 1 bis 8, das ferner das Verabreichen einer oder mehrerer Dosen eines Inhibitors des Proprotein-Convertase-Subtilisin-Kexin-9 (PCSK9) an das Subjekt umfasst, wobei der PCSK9-Inhibitor ein Antikörper oder ein antigenbindendes Fragment davon ist, der/das spezifisch PCSK9 bindet.

10. ANGPTL3-Inhibitor für die Verwendung in dem Verfahren nach Anspruch 9, wobei der Antikörper oder das antigenbindende Fragment davon, der/das spezifisch PCSK9 bindet, ausgewählt ist, aus der Gruppe, bestehend aus Alirocumab, Evolocumab, Bococizumab, Lodelcizumab und Ralpancizumab.

11. ANGPTL3-Inhibitor für die Verwendung in dem Verfahren nach einem der Ansprüche 1 bis 10, das ferner das Verabreichen einer oder mehrerer Dosen eines ANGPTL4-Inhibitors und/oder ANGPTL8-Inhibitors an das Subjekt umfasst, wobei der ANGPTL4-Inhibitor ein Antikörper oder ein antigenbindendes Fragment davon ist, der/das spezifisch ANGPTL4 bindet, wobei der ANGPTL8-Inhibitor ein Antikörper oder ein antigenbindendes Fragment davon ist, der/das spezifisch ANGPTL8 bindet.

12. Pharmazeutische Zusammensetzung für die Verwendung in einem Verfahren zum Reduzieren des Fortschreitens von Atherosklerose in einem Subjekt, wobei die Zusammensetzung einen Inhibitor des Angiopoietin-ähnlichen Proteins 3 (ANGPTL3) umfasst, und wobei eine oder mehrere Dosen des Inhibitors des Angiopoietin-ähnlichen Proteins 3 (ANGPTL3) an das Subjekt verabreicht werden, wobei der ANGPTL3-Inhibitor ein Antikörper oder ein antigenbindendes Fragment davon ist, der/das spezifisch ANGPTL3 bindet, der die CDR-Aminosäuresequenzen der schweren und leichten Kette mit den SEQ ID NO: 4, 5, 6, 7, 8 und 9 umfasst.

## Revendications

1. Inhibiteur de protéine angiopoïétine de type 3 (ANGPTL3) destiné à être utilisé dans un procédé de réduction de la progression d'athérosclérose chez un sujet, le procédé comprenant la sélection d'un sujet qui est atteint d'athérosclérose, et l'administration d'une ou de plusieurs doses de l'inhibiteur de protéine angiopoïétine de type 3 au sujet, dans lequel l'inhibiteur de ANGPTL3 est un anticorps, ou fragment de liaison à l'antigène de celui-ci, qui se lie spécifiquement à ANGPTL3, qui comprend des séquences d'acides aminés de CDR de chaînes lourde et légère ayant SEQ ID n° : 4, 5, 6, 7, 8, et 9.

2. Inhibiteur de ANGPTL3 destiné à être utilisé dans le procédé selon la revendication 1, dans lequel, avant ou à l'instant du commencement du traitement avec l'une ou les plusieurs doses de l'inhibiteur de ANGPTL3, il est découvert que le sujet est atteint d'hypercholestérolémie familiale hétérozygote (HFHe) ou d'hypercholestérolémie familiale homozygote (HFHo), et/ou de lipoprotéine(a) élevée (Lp[a]).

3. Inhibiteur de ANGPTL3 destiné à être utilisé dans le procédé selon la revendication 1, dans lequel, avant ou à l'instant du commencement du traitement avec l'une ou les plusieurs doses de l'inhibiteur de ANGPTL3, il est découvert que le sujet est atteint d'une maladie cardiovasculaire (MCV), ou a souffert d'un accident vasculaire cérébral ou d'un infarctus du myocarde.

4. Inhibiteur de ANGPTL3 destiné à être utilisé dans le procédé selon l'une quelconque des revendications 1 à 3, dans lequel le sujet est traité avec une thérapie de modification des lipides (LMT) de base stable avant ou à l'instant du commencement du traitement avec l'une ou les plusieurs doses de l'inhibiteur de ANGPTL3, ou simultanément à l'administration de l'une ou des plusieurs doses de l'inhibiteur de ANGPTL3, optionnellement dans lequel la LMT de base stable est une thérapie par statine à dose faible, modérée, ou haute.

5. Inhibiteur de ANGPTL3 destiné à être utilisé dans le procédé selon l'une quelconque des revendications 1 à 4, dans lequel l'administration de l'une ou des plusieurs doses de l'inhibiteur de ANGPTL3 au sujet a pour résultat une ou plusieurs conséquence(s) thérapeutique(s) sélectionnées parmi le groupe constitué de :
(a) une réduction du niveau de cholestérol total (CT) sérique,
(b) une réduction du niveau de triglycéride (TG) sérique,
(c) une réduction du niveau de lipoprotéine de basse densité (LDL) sérique, et
(d) une réduction du niveau de lipoprotéine de très basse densité (VLDL) sérique ;
dans lequel la réduction de (a), de (b), de (c) et/ou de (d) est déterminée relativement au niveau de TC sérique, au niveau de TG sérique, au niveau de LDL sérique, et/ou au niveau de VLDL sérique du sujet avant ou à l'instant du commencement du traitement avec l'une ou les plusieurs doses de l'inhibiteur de ANGPTL3.

6. Inhibiteur de ANGPTL3 destiné à être utilisé dans le procédé selon l'une quelconque des revendications 1 à 5, dans lequel l'administration de l'une ou des plusieurs doses de l'inhibiteur de ANGPTL3 au sujet a pour résultat une réduction de formation de plaques athérosclérotiques.

7. Inhibiteur de ANGPTL3 destiné à être utilisé dans le procédé selon l'une quelconque des revendications précédentes, dans lequel l'anticorps, ou fragment de liaison à l'antigène de celui-ci, qui se lie spécifiquement à ANGPTL3 comprend une HCVR ayant la séquence d'acides aminés de SEQ ID n° : 2 et une LCVR ayant la séquence d'acides aminés de SEQ ID n° : 3.

8. Inhibiteur de ANGPTL3 destiné à être utilisé dans le procédé selon la revendication 1, dans lequel l'anticorps, ou fragment de liaison à l'antigène de celui-ci, qui se lie spécifiquement à ANGPTL3 est évinacumab.

9. Inhibiteur de ANGPTL3 destiné à être utilisé dans le procédé selon l'une quelconque des revendications 1 à 8, comprenant en outre l'administration d'une ou de plusieurs doses d'un inhibiteur de proprotéine convertase subtilisine/kexine-9 (PCSK9) au sujet, dans lequel l'inhibiteur de PCSK9 est un anticorps, ou fragment de liaison à l'antigène de celui-ci, qui se lie spécifiquement à PCSK9.

10. Inhibiteur de ANGPTL3 destiné à être utilisé dans le procédé selon la revendication 9, dans lequel l'anticorps, ou le fragment de liaison à l'antigène de celui-ci, qui se lie spécifiquement à PCSK9 est sélectionné parmi le groupe constitué de : alirocumab, évolocumab, bococizumab, lodelcizumab, et ralpancizumab.

11. Inhibiteur de ANGPTL3 destiné à être utilisé dans le procédé selon l'une quelconque des revendications 1 à 10, comprenant en outre l'administration d'une ou de plusieurs doses d'un inhibiteur de ANGPTL4 et/ou d'un inhibiteur de ANGPTL8 au sujet, dans lequel l'inhibiteur de ANGPTL4 est un anticorps, ou fragment de liaison à l'antigène de celui-ci, qui se lie spécifiquement à ANGPTL4, dans lequel l'inhibiteur de ANGPTL8 est un anticorps, ou fragment de liaison à l'antigène de celui-ci, qui se lie spécifiquement à ANGPTL8.

12. Composition pharmaceutique destinée à être utilisée dans un procédé de réduction de la progression d'athérosclérose chez un sujet, dans lequel la composition comprend un inhibiteur de protéine angiopoïétine de type 3 (ANGPTL3), et dans lequel une ou plusieurs doses de l'inhibiteur de protéine angiopoïétine de type 3 (ANGPTL3) sont administrées au sujet, dans lequel l'inhibiteur de ANGPTL3 est un anticorps, ou fragment de liaison à l'antigène de celui-ci, qui se lie spécifiquement à ANGPTL3 qui comprend des séquences d'acides aminés de CDR de chaînes lourde et légère ayant SEQ ID n° : 4, 5, 6, 7, 8, et 9.
